(19) Europäisches Patentamt / European Patent Office / Office européen des brevets

(11) **EP 2 814 524 B1**

(12) **EUROPEAN PATENT SPECIFICATION**

(45) Date of publication and mention
of the grant of the patent:
**11.12.2019 Bulletin 2019/50**

(51) Int Cl.:
*A61L 27/18* *(2006.01)* *A61L 27/20* *(2006.01)*
*A61L 27/56* *(2006.01)* *A61L 15/26* *(2006.01)*
*A61L 15/42* *(2006.01)*

(21) Application number: **13712391.5**

(22) Date of filing: **15.02.2013**

(86) International application number:
**PCT/PL2013/000020**

(87) International publication number:
**WO 2013/122492 (22.08.2013 Gazette 2013/34)**

(54) **MEDICAL MATERIAL, THE METHOD OF ITS PRODUCTION AND USE OF THE MEDICAL MATERIAL**

MEDIZINISCHES MATERIAL, VERFAHREN ZU DESSEN HERSTELLUNG UND VERWENDUNG DES MEDIZINISCHEN MATERIALS

MATIÈRE MÉDICALE, SON PROCÉDÉ DE FABRICATION ET UTILISATION DE LA MATIÈRE MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.02.2012 PL 39813912**

(43) Date of publication of application:
**24.12.2014 Bulletin 2014/52**

(73) Proprietor: **Politechnika Lodzka**
**90-924 Lodz (PL)**

(72) Inventors:
• **KRUCINSKA, Izabella**
**91-857 Lódz (PL)**
• **KOMISARCZYK, Agnieszka**
**91-473 Lódz (PL)**

• **KOWALSKA, Stanislawa**
**91-372 Lódz (PL)**
• **CHRZANOWSKI, Mìcha**
**91-520 Lódz (PL)**
• **STRUSZCZYK, Marcìn**
**95-070 Ruda Bugaj (PL)**

(74) Representative: **Twardowska, Aleksandra**
**Kulikowska & Kulikowski**
**Roma Office Center**
**ul.Nowogrodzka 47A**
**00-695 Warszawa (PL)**

(56) References cited:
**EP-A1- 1 216 717** **WO-A1-01/02033**
**WO-A1-2011/038084** **DE-A1-102005 042 707**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 814 524 B1

**Description**

The invention claimed concerns a medical material, the method of its production, as well as use of that medical material manufactured from dibutyrylchitin and chitin regenerated from dibutyrylchitin, butyrylchitin esterified to various degrees or from a mixture of dibutyrylchitin and polylactide. The solution concerns a new method of biodegradable polymer foam formation. Biodegradable polymers covered by the scope of this patent application undergo degradation processes as a result of presence of at least one of a number of physical and/or chemical factors.

The relevant literature provides information concerning the methods of obtaining foams of various polymer types: polyurethanes (US 2011034575), methacrylates (US 2011031435), polyesters (WO2009149845) and others.

To create a porous structure, aerogels (WO2011016962), silicas (US 2011034571) or blowing agents with unidentified chemical structure are used. Depending on the type of the blowing agent used, the pores of the obtained foams are either open or closed pores. The production of foams is based mainly on polymer melts.

Patent application WO 2011015568 describes the method of production of polyurethane foams to be used as resorbable dressing materials characterized by increased absorbency.

Some composite materials in which foams constitute at least one of the elements have also been developed (WO 2011015299) EP1216717 A discloses a bioabsorbable tissue implant comprising a polymeric foam and a reinforcing polymeric mesh. In Example 1, a solution of PCL/PLA is added to a PGA/PLA mesh.

Despite the solutions existing to date, none of the available documents includes the use of dibutyrylchitin or a mixture of dibutyrylchitin and polylactide, possessing favorable biodegradability properties, addressed by the presented solution. Similarly, none of the available documents includes the use of the claimed polymer mixture solution as a matrix.

The aim of the presented invention is to provide a solution utilizing foam-based materials as medical products making it possible to enhance the wound healing processes, characterized at the same time by biodegradability as a result of contact with a living organism, and allowing to regulate the biodegradation time, as medical products for wound therapy, implantable materials, scaffolds for cell cultures, as well as components of composites designed for the aforementioned applications.

The aim of the presented invention is to provide the agents which could be used for production of composite medical materials, in which at least one of the components is dibutyrylchitin or butyrylchitin esterified to various degrees, in order to obtain medical products characterized by biodegradability in contact with the organism.

The realization of an objective defined as above and solution of the problems described in the state of the art, associated with obtaining medical materials allowing to improve the healing processes, characterized at the same time by biodegradability as a result of contact with the organism and making it possible to control the biodegradation time to be used as medical products for wound therapy, implantable materials, scaffolds for cell cultures, as well as components of composites designed for the aforementioned applications has been achieved within the framework of this invention. One subject of the invention is a medical material containing a foam mass composition consisting of a mixture of dibutyrylchitin and polylactide, wherein surface mass is 208 g/m$^2$, the test liquid A absorbency is CA = 12.748 $g_{liquid}$ /$g_{sampie}$ and free water sorption in time t = 8h is 10.600 $g_{liquid}$ /$g_{sampie}$ and wherein foam mass composition is obtained by mixing a PLA solution in acetone of 5% w/w concentration with a DBC solution in acetone of a 5% w/ w concentration with addition of a plasticizer which is glycerol in the amount of 5% w/w as related to the polymer mixture solution mass and a blowing agent which is solid sodium chloride in the amount of 150% w/w as related to polymer mixture solution mass.

Preferably, a foamy product is combined with a fibrous product.

Preferably, the material is a dressing and/or implantable material, and/or a material used in tissue engineering. Preferably, the material constitutes an element of an implantable composite. Preferably, the material constitutes a scaffold for tissue culture. Preferably, the material is designed for wound dressing. Preferably, the material is designed for use as a product with prophylactic properties. Preferably, the material is saturated with an antibacterial, antiinflammatory, antipyretic, analgesic agent, an anticoagulant or a drug accelerating proliferation of cells.

Another subject of the invention is a medical material containing foam mass composition comprising dibutyrylchitin wherein surface mass is 864.2 g/m$^2$ and free water sorption in time t=8h is 4.3 $g_{liquid}$ /$g_{sampie}$ and wherein foam mass composition is obtained by mixing DBC solution in ethanol 8% w/w with addition of a blowing agent which is sodium chloride in amount 100% of polymer solution mass and wherein the obtained mass was poured onto a surface of needled nonwoven made of DBC fibers of 240 g/m$^2$ surface mass.

Preferably, the foam is combined with any substrate in the form of nonwoven, foam, film, gel or hydrogel, the method of foam combination with any textile material involving: a) application of the liquid foam mass in accordance with the invention onto the surface of the coated material, on its one or both sides, and then its solidification, or b) saturation of the material with the liquid foam mass in accordance with the invention, or c) binding of the solidified foam in accordance with the invention using a polymer adhesive approved for medical applications.

Preferably, the material is impregnated with any drug.

The next subject of the invention is the application of the material described above in manufacturing medical products.

The products covered by the scope of the invention serve for obtaining a medical product of any size and form. The term "medical product" is understood as dressing materials, implantable materials and scaffolds for tissue cultures, as well as elements of composite materials designed for use in medicine.

Preferably, the dressing protects the surface of wounds, especially against excess fluid loss via the extrarenal route.

For better illustration of the invention, the solutions have been presented in figures, where:

**figure 1** presents a cross-section of the formed foams - SEM images of PLA foam - example 1;

**figure 2** presents a cross-section of the formed foams - SEM images of PLA foam - example 2;

**figure 3** presents a cross-section of the formed foams - SEM images of PLA co-GA foam - example 3;

**figure 4** presents the structure of PLA foam with dibutyrylchitin - obtained foams - example 4;

**figure 5** presents the structure of PLA foam with polyethylene glycol - obtained foams - example 5;

**figure 6** presents the structure of PLA foam with polyethylene glycol and sodium chloride - obtained foams - example 6;

**figure 7** presents the structure of PLA foam with polyethylene glycol and sodium chloride - obtained foams - example 7;

**figure 8** presents the structure of PLA foam with carboxymethylcellulose and glycerol - obtained foams - example 8;

**figure 9** presents the structure of PLA foam with sodium alginate and glycerol - obtained foams - example 9;

**figure 10** presents the view of foam formed from PLA melt - example 10;

**figure 11** presents the structure of a composite in which the foam mass coats the surface of the fibrous layer - example 11;

**figure 12** presents the structure of PLA foam/PLA fiber composite - obtained polymer composites from example 13;

**figure 13** presents the structure of PLA foam/PLA fiber composite - obtained polymer composites from example 14;

**figure 14** presents the structure of PLA foam with a drug (chlorhexidine) - example 15;

**figure 15** presents the kinetic curves of drug release into water and into physiological saline solution - example 15;

**figure 16** presents the structure of PLA foam with PLA film - example 16;

**figure 17** presents the structure of PLA foam with PLA film - example 17;

**figure 18** presents the structure of PLA foam bound to the surface of spun-bonded nonwoven - example 18.

For better understanding of the invention, examples of processing have been presented below.

## Examples

From the following examples, only examples 4, 11, 12, 17, and 18 fall within the scope of the appended claims. The other examples are shown for reference purposes only.

In all the examples, the test liquid A according to EN13726-1:2005 was prepared as a solution of 8.298 g NaCl + 0.368 g $CaCl_2$ x $2H_2O$ in 1 liter $H_2O$.

### Example 1

Foam mass composition:

PLA solution Mw = 87 000 g/mol in ethyl acetate of 10% w/w concentration

Plasticizer - glycerol in the amount of 5% w/w as related to PLA solution mass.

Blowing agent - solid sodium chloride in the amount of 100% w/w as related to PLA solution mass.

The components were mixed in a vessel non-reactive with any of the components. Mixing was effected by means of a stirrer with a flexible shaft. The obtained mass was poured onto a surface of dimensions and shape of the desired foam. Evaporation of ethyl acetate took place at ambient temperature for the period of ca. 10 hours, up to the moment that complete, free detachment of the foam product from the substrate was possible.

Then the foaming agent (sodium chloride) was washed out with running water. The last rinse was carried out with deionized water. Rinsing was continued up to the disappearance of the reaction characteristic of chloride ions with silver nitrate. As a result, a foamy material with open pores and the following parameters was obtained:

Surface mass: 121.5 $g/m^2$,

Test liquid A absorbency according to EN13726-1:2005: $C_A$= 9.798 $g_{liquid}/g_{sample}$,

Free water sorption in time t=8h according to EN13726-1:2005: 10.193 $g_{liquid}/g_{sample}$,

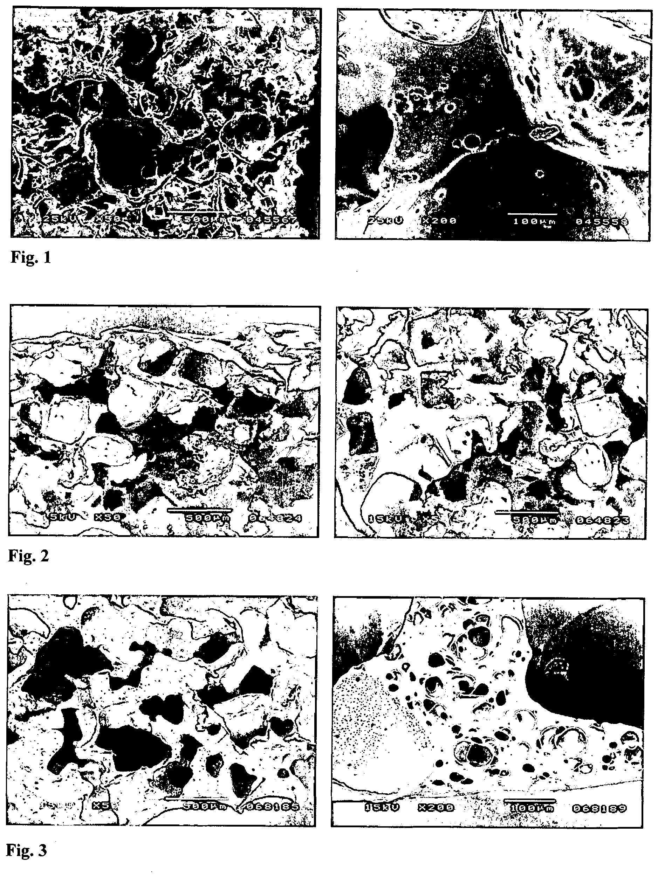

The cross-sectional view of the formed foams is presented in figure 1.

## Example 2

Foam mass composition:

PCL solution, Mw=60 000 g/mol in dichloromethane of 13% w/w concentration
Plasticizer - glycerol in the amount of 2.5% w/w as related to PCL solution mass.
Blowing agent - solid sodium chloride in the amount of 150% w/w as related to PCL solution mass.

The components were mixed in a vessel non-reactive with any of the components. Mixing was effected by means of a stirrer with a flexible shaft. The obtained mass was poured onto a surface of dimensions and shape of the desired foam. Evaporation of dichloromethane took place at ambient temperature for the period of ca. 8 hours, up to the moment that complete, free detachment of the foam product from the substrate was possible.
Then the foaming agent (sodium chloride) was washed out with running water. The last rinse was carried out with deionized water. Rinsing was continued up to the disappearance of the reaction characteristic of chloride ions with silver nitrate. As a result, a foamy material with open pores and the following parameters was obtained:

Surface mass: 121.5 g/m$^2$,
Test liquid A absorbency according to EN13726-1:2005: $C_A$= 6.924 $g_{liquid}/g_{sample}$,
Free water sorption in time t=8h according to EN13726-1:2005: 8.335 $g_{liquid}/g_{sample}$,
The cross-sectional view of the formed foams is presented in figure 2.

## Example 3

Foam mass composition:

PLA co - GA solution Mw = 200 000 g/mol in dichloromethane of 10% w/w concentration. PLA co - GA had composition 79:21 to 85:15 molar ratio of L - Lactide:glycolide, delivered by Evonik Rohm GmbH
Plasticizer - glycerol in the amount of 5% w/w as related to PLA co - GA solution mass.
Blowing agent - solid sodium chloride in the amount of 75% w/w as related to PLA co - GA solution mass.

The components were mixed in a vessel non-reactive with any of the components. Mixing was effected by means of a stirrer with a flexible shaft. The obtained mass was poured onto a surface of dimensions and shape of the desired foam. Evaporation of dichloromethane took place at ambient temperature for the period of ca. 8 hours, up to the moment that complete, free detachment of the foam product from the substrate was possible.
Then the foaming agent (sodium chloride) was washed out with running water. The last rinse was carried out with deionized water. Rinsing was continued up to the disappearance of the reaction characteristic of chloride ions with silver nitrate. As a result, a foamy material with open pores and the following parameters was obtained:

Surface mass: 235.3 g/m$^2$,
Test liquid A absorbency according to EN13726-1:2005: $C_A$= 7.6 $g_{liquid}/g_{sample}$,
Free water sorption in time t=8h according to EN13726-1:2005: 7.4 $g_{liquid}/g_{sample}$,
The cross-sectional view of the formed foams is presented in figure 3.

## Example 4

Foam mass composition:

PLA solution Mw = 87 000 g/mol in acetone of 5% w/w concentration.
DBC solution in acetone of 5% w/w concentration.
Plasticizer - glycerol in the amount of 5% w/w as related to the polymer mixture solution mass.
Blowing agent - solid sodium chloride in the amount of 150% w/w as related to polymer mixture solution mass.

Polymer solutions were mixed. A plasticizing agent and a blowing agent were added to the mixture. The components were mixed in a vessel non-reactive with any of the components. Mixing was effected by means of a stirrer with a flexible shaft. The obtained mass was poured onto a surface of dimensions and shape of the desired foam. Evaporation of acetone took place at ambient temperature for the period of ca. 10 hours, up to the moment that complete, free detachment of the foam product from the substrate was possible.
Then the foaming agent (sodium chloride) was washed out with running water. The last rinse was carried out with deionized water. Rinsing was continued up to the disappearance of the reaction characteristic of chloride ions with silver nitrate. As a result, a foamy material with open pores and the following parameters was obtained:

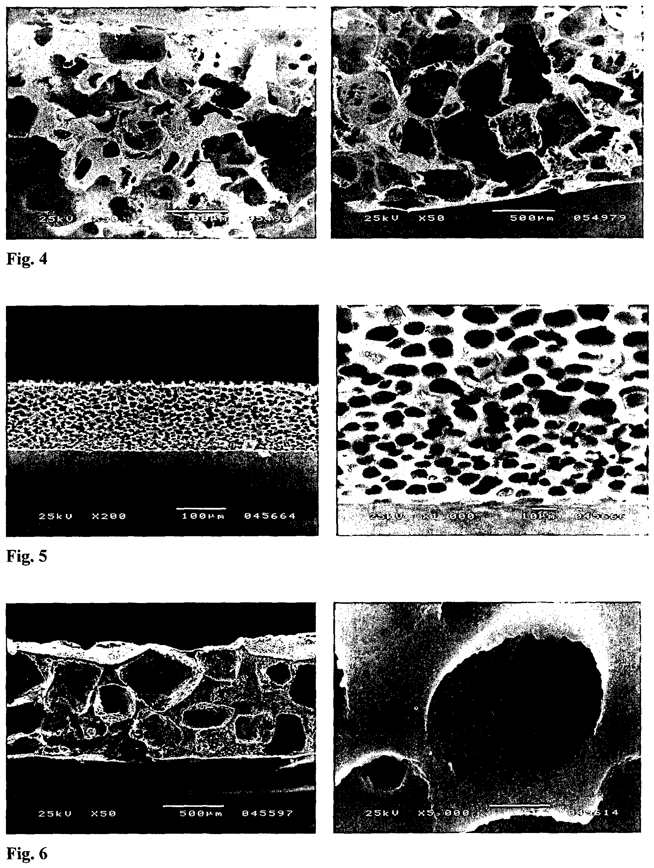

Surface mass: 208 g/m$^2$,
Test liquid A absorbency according to EN13726-1:2005: $C_A$= 12.748 g$_{liquid}$/g$_{sample}$,
Free water sorption in time t=8h according to EN13726-1:2005: 10.600 g$_{liquid}$/g$_{sample}$,
The obtained foams are presented in figure 4.

**Example 5**

Foam mass composition:

PLA solution Mw = 87 000 g/mol in ethyl acetate of 10% w/w concentration.
Blowing agent and plasticizer - polyethylene glycol of molecular weight Mw = 400 g/mol. - 10% w/w as related to the polymer solution mass.

The components were mixed in a vessel non-reactive with any of the components. Mixing was effected by means of a stirrer with a flexible shaft. The obtained mass was poured onto a surface of dimensions and shape of the desired foam. Evaporation of PLA solvent - ethyl acetate - took place at ambient temperature for the period of ca. 10 hours, up to the moment that complete, free detachment of the foam product from the substrate was possible.
Then the excess polyethylene glycol was removed from the obtained foam with blotting paper or a paper towel.
As a result, a foamy material with open pores and the following parameters was obtained:

Surface mass: 300.7 g/m$^2$,
Test liquid A absorbency according to EN13726-1:2005: $C_A$= 0.33 g$_{liquid}$/g$_{sample}$,
Free water sorption in time t=8h according to EN13726-1:2005: 0.738 g$_{liquid}$/g$_{sample}$,
The obtained foams are presented in figure 5.

**Example 6**

Foam mass composition:

PLA solution Mw = 87 000 g/mol in ethyl acetate of 10% w/w concentration.

Plasticizer - polyethylene glycol of molecular weight Mw = 400 g/mol. - 10% w/w as related to the polymer solution mass. Blowing agent - solid sodium chloride in the amount of 100% w/w as related to polymer solution mass.

The components were mixed in a vessel non-reactive with any of the components. Mixing was effected by means of a stirrer with a flexible shaft. The obtained mass was poured onto a surface of dimensions and shape of the desired foam. Evaporation of PLA solvent - ethyl acetate - took place at ambient temperature for the period of ca. 10 hours, up to the moment that complete, free detachment of the foam product from the substrate was possible. Then the excess polyethylene glycol was removed from the obtained foam with blotting paper or a paper towel.
Then the foaming agent (sodium chloride) was washed out with running water. The last rinse was carried out with deionized water. Rinsing was continued up to the disappearance of the reaction characteristic of chloride ions with silver nitrate. As a result, a foamy material with open pores and the following parameters was obtained:

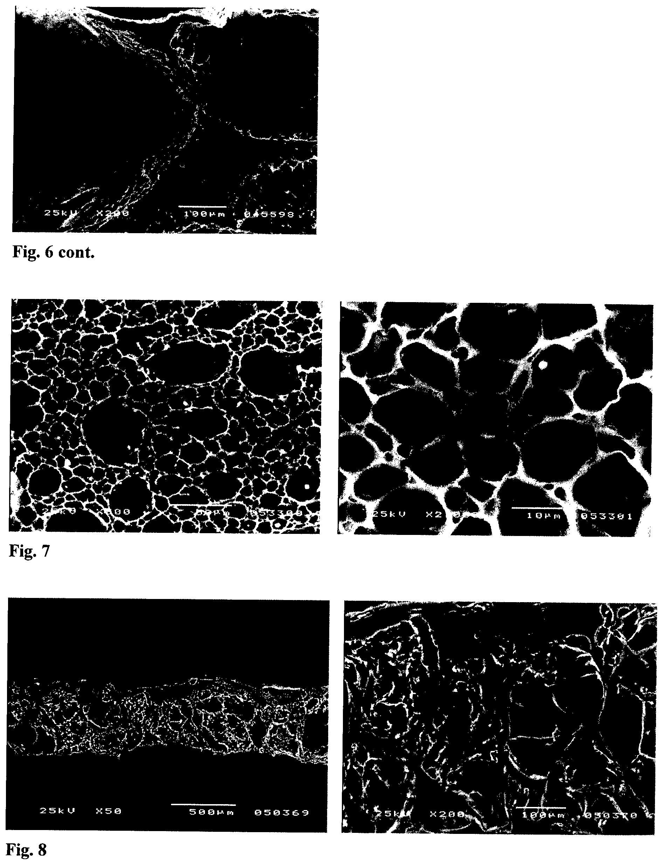

Surface mass: 117.3 g/m$^2$,
Test liquid A absorbency according to EN13726-1:2005: $C_A$= 1.78 g$_{liquid}$/g$_{sample}$,
Free water sorption in time t=8h according to EN13726-1:2005: 3.1 g$_{liquid}$/g$_{sample}$,
The obtained foams are presented in figure 6.

**Example 7**

Foam mass composition:

PLA solution Mw = 87 000 g/mol in ethyl acetate of 10% w/w concentration.
Plasticizer - polyethylene glycol of molecular weight Mw = 400 g/mol. - 20% w/w as related to the polymer solution mass. Blowing agent - solid sodium chloride in the amount of 100% w/w as related to polymer solution mass.

The components were mixed in a vessel non-reactive with any of the components. Mixing was effected by means of a stirrer with a flexible shaft. The obtained mass was poured onto a surface of dimensions and shape of the desired foam. Evaporation of PLA solvent - ethyl acetate - took place at ambient temperature for the period of ca. 10 hours, up to the moment that complete, free detachment of the foam product from the substrate was possible. Then the excess polyethylene glycol was removed from the obtained foam with blotting paper or a paper towel.

Then the foaming agent (sodium chloride) was washed out with running water. The last rinse was carried out with deionized water. Rinsing was continued up to the disappearance of the reaction characteristic of chloride ions with silver nitrate. As a result, a foamy material with open pores and the following parameters was obtained:

Surface mass: 407.6 $g/m^2$,
Test liquid A absorbency according to EN13726-1:2005: $C_A$= 4.930 $g_{liquid}/g_{sample}$,
Free water sorption in time t=8h according to EN13726-1:2005: 4.121 $g_{liquid}/g_{sample}$,
The obtained foams are presented in figure 7.

**Example 8**

Foam mass composition:

PLA solution Mw = 87 000 g/mol in ethyl acetate 10% w/w.
Carboxymethylcellulose in solid form 10% w/w as related to PLA solution mass.
Plasticizer - glycerol in the amount of 5% w/w as related to the polymer solution mass.

The components were mixed in a vessel non-reactive with any of the components. Mixing was effected by means of a stirrer with a flexible shaft. The obtained mass was poured onto a surface of dimensions and shape of the desired foam. Evaporation of PLA solvent - ethyl acetate - took place at ambient temperature for the period of ca. 10 hours, up to the moment that complete, free detachment of the foam product from the substrate was possible. As a result, a foamy material with open pores and the following parameters was obtained:

Surface mass: 794.21 $g/m^2$,
Free water sorption in time t=8h according to EN13726-1:2005: 3.40 $g_{liquid}/g_{sample}$,
Test liquid A absorbency according to EN13726-1:2005: 3.23 $g_{liquid}/g_{sample}$,
The obtained foams are presented in figure 8.

**Example 9**

Foam mass composition:

PLA solution Mw = 87 000 g/mol in ethyl acetate 10% w/w.
Sodium alginate in the amount of 10% w/w as related to PLA solution mass.
Plasticizer - glycerol in the amount of 5% w/w as related to the polymer solution mass.

The components were mixed in a vessel non-reactive with any of the components. Mixing was effected by means of a stirrer with a flexible shaft. The obtained mass was poured onto a surface of dimensions and shape of the desired foam. Evaporation of PLA solvent - ethyl acetate - took place at ambient temperature for the period of ca. 10 hours, up to the moment that complete, free detachment of the foam product from the substrate was possible. As a result, a foamy material with open pores and the following parameters was obtained:

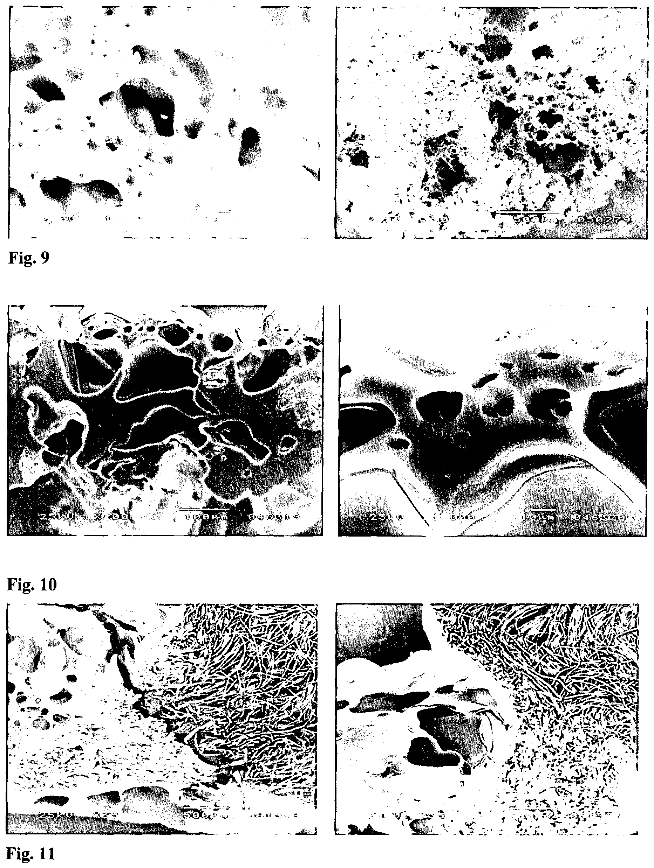

Surface mass: 620.4 $g/m^2$,
Test liquid A absorbency according to EN13726-1:2005: $C_A$= 2.24 $g_{liquid}/g_{sample}$,
Free water sorption in time t=8h according to EN13726-1:2005: 2.80 $g_{liquid}/g_{sample}$,
The obtained foams are presented in figure 9.

**Example 10**

```
Polymer - PLA Mw = 87 000 g/mol
```

Plasticizer - polyethylene glycol of molecular weight Mw = 6000 g/mol. - 10% w/w as related to the polymer solution mass.

Blowing agent - sodium bicarbonate $NaHCO_3$ 30% w/w as related to the polymer mass.

The compounds in solid form were mixed thoroughly. The obtained mixture was placed in the hopper of a Mini - Lab laboratory extruder (Haake, Germany). Extrusion conditions: temperature T=180°C, rotary speed of the screws 50 rpm. The mass was extruded forming into the desired shape. The extruded mass was solidified by cooling the molten polymer in air at ambient temperature.

As a result, foamy products presented in figure 10 were obtained.

## Example 11

Foam mass composition:

DBC solution in ethanol 8% w/w.

Blowing agent - sodium chloride - 100% of polymer solution mass

The components were mixed in a vessel non-reactive with any of the components. Mixing was effected by means of a stirrer with a flexible shaft. The obtained mass was poured onto a surface of needled nonwoven made of DBC fibers of 240 g/m² surface mass. Ethyl alcohol was removed by coagulation in coagulating water bath with 5% of ethanol added. As a result, a foamy material with open pores coating the nonwoven surface was obtained. Due to the effect of the solvent, the fibers were partially dissolved, forming a binding layer. The composite structure is presented in figure 11.

Surface mass: 864.2 g/m²,

Free water sorption in time t=8h according to EN13726-1:2005: 4.3 $g_{liquid}/g_{sample}$,

## Example 12

Composite from example 11 can be subjected to hydrolysis in caustic soda to obtain chitin regenerated from dibutyrylchitin or butyrylchitin esterified to various degrees. Hydrolysis conditions:

Hydrolyzing bath containing 5% of sodium hydroxide (NaOH). Hydrolysis was conducted at 25°C for 2 h.

Shortening the hydrolysis time led to obtaining butyrylchitin of esterification grade between 0 and 2.

After hydrolysis, the product was rinsed five times with distilled water until neutral pH was obtained.

## Example 13

The foam mass obtained according to example 1 was applied onto the surface of a layer of unbound PLA fibers of linear mass Ttex = 2.81 dtex. Evaporation of PLA solvent - ethyl acetate - took place at ambient temperature for the period of ca. 10 hours, up to the moment that complete, free detachment of the foam product from the substrate was possible. As a result, a foamy material with open pores penetrating the whole structure of the fibrous layer, characterized by the following parameters, was obtained:

Surface mass: 460 g/m²,

Test liquid A absorbency according to EN13726-1:2005: $C_A$= 2.34 $g_{liquid}/g_{sample}$,

Free water sorption in time t=8h according to EN13726-1:2005: 3.90 $g_{liquid}/g_{sample}$,

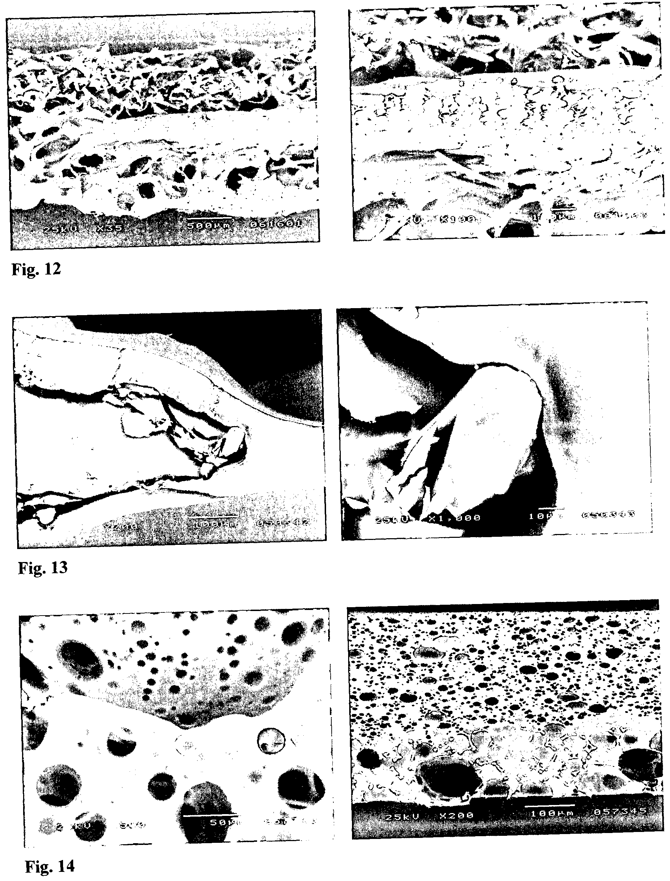

The obtained foam composites are presented in figure 12.

## Example 14

The foam mass obtained according to example 1 was applied onto the surface of hydrogel in a dry form. Evaporation of PLA solvent - ethyl acetate - took place at ambient temperature for the period of ca. 10 hours, up to the moment that complete, free detachment of the foam product from the substrate was possible. As a result, a polymer material embedding the hydrogel structures was obtained. In contact with a liquid, the liquid is absorbed and development of the hydrogel surface takes place.

Surface mass: 789 g/m²,

Test liquid A absorbency according to EN13726-1:2005 and free water sorption in time t=8h according to EN13726-1:2005: could not be determined because under the measurement conditions the hydrogel swells and is detached from the matrix.

The obtained polymer composites are presented in figure 13.

**Example 15**

Foam mass composition:

PLA solution Mw = 87 000 g/mol in ethyl acetate 10% w/w.
Plasticizer - glycerol in the amount of 5% w/w as related to PLA solution mass.

Aditional substances: Chlorhexidine (1,6-di-(4-chlorophenylbiguanido)-hexane 1,6-di-(4-chlorophenylbiguanido)-hexane) in the amount of 2.5% as related to the polymer solution mass.

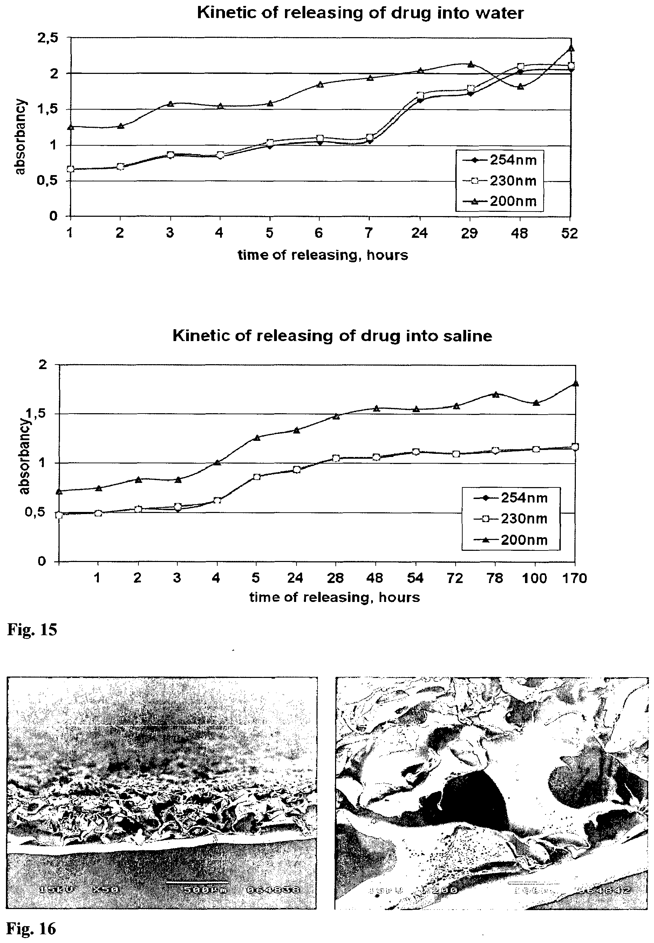

The components were mixed in a vessel non-reactive with any of the components. Mixing was effected by means of a stirrer with a flexible shaft. The obtained mass was poured onto a surface of dimensions and shape of the desired foam. Evaporation of ethyl acetate took place at ambient temperature for the period of ca. 10 hours, up to the moment that complete, free detachment of the foam product from the substrate was possible. After evaporation of the solvent and solidification of the foam, the weighed samples of foam with the drug were prepared. The drug was released into water and physiological saline at 37°C using a laboratory shaker. The kinetics of release (release time 1 - 52 h in water and 1 - 194 h in physiological saline) was analyzed by measuring UV radiation absorbance with a JASCO V-670 spectrophotometer. The calculations were carried aut on the basis of the calibration curve determined for the drug. Absorbance was read for three bands: 254 nm, 230 nm, 200 nm, which are characteristic of the drug.
Surface mass: 390 g/m$^2$,
Test liquid A absorbency according to EN13726-1:2005: CA= 2.96 $g_{liquid}/g_{sample}$,
Free water sorption in time t=8h according to EN13726-1:2005: 3.48 $g_{liquid}/g_{sample}$,
The structure of PLA foam with the drug (chlorhexidine) is presented in figure 14, whereas the kinetic curves of drug release into water and into physiological saline are presented in figure 15.

**Example 16**

The foam obtained according to example 1 was permanantly combined with PLA film Mw = 87 000 g/mol of 0.05 mm thickness formed with the melt technique. The binding method involved welding the film with the foam at 120°C temperature for 5 s with heating plate pressure of 2.5 atm.
Surface mass: 345 g/m$^2$,
Test liquid A absorbency according to EN13726-1:2005: CA= 2.00 $g_{liquid}/g_{sample}$,
Free water sorption in time t=8h according to EN13726-1:2005: 3.26 $g_{liquid}/g_{sample}$,
The structure of PLA foam with PLA film is presented in figure 16.

**Example 17**

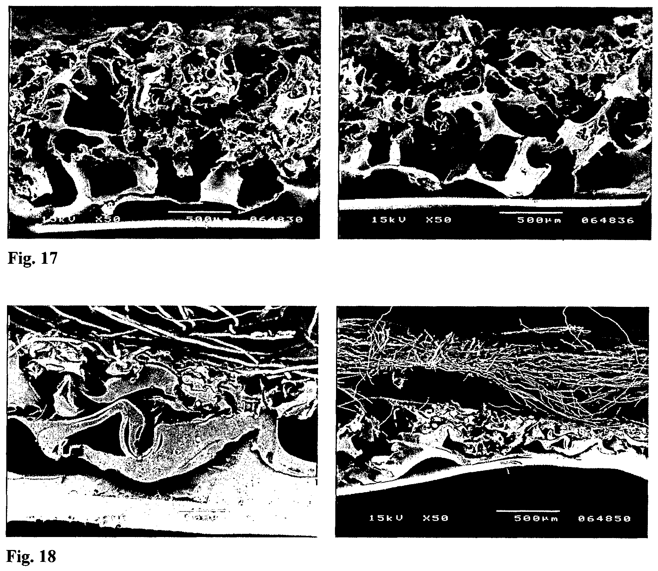

Foam from example 4 was permanently combined with PLA film Mw = 87 000 g/mol of 0.05 mm thickness formed with the melt technique. The binding method involved welding the film with the foam at 120°C temperature for 5 s without heating plate pressure.
Surface mass: 350 g/m$^2$,
Test liquid A absorbency according to EN13726-1:2005: CA= 4.60 $g_{liquid}/g_{sample}$,
Free water sorption in time t=8h according to EN13726-1:2005: 5.73 $g_{liquid}/g_{sample}$,
The structure of PLA foam with PLA film is presented in figure 17.

**Example 18**

Foam from example 4 was combined with spun-bonded PLA nonwoven of 20 g/m$^2$ surface mass. The binding method involved welding the film with the foam at 120°C temperature for 5 s without heating plate pressure.
Surface mass: 382 g/m$^2$,
Test liquid A absorbency according to EN13726-1:2005: CA= 3.50 $g_{liquid}/g_{sample}$,
Free water sorption in time t=8h according to EN13726-1:2005: 4.22 $g_{liquid}/g_{sample}$,
The structure of PLA foam bound to the surface of spun-bonded nonwoven is presented in figure 18.

**Example 19**

Calcium alginate microspheres containing insulin-like growth factor IGF-1 (1376 Sigma-Aldrich product) were prepared. 3.5% sodium alginate solution in 100 ml was used to obtain the microspheres. 50μg IGF-1 was added to the solution. The microspheres were produced on a laboratory stand for microspheres and microcapsules formation, under technological air pressure P=0.3atm. The microspheres were solidified on aqueous calcium chloride solution $CaCl_2$ $X2H_2O$ of 5% concentration. Then, the obtained microspheres were rinsed with distilled water and dried at ambient temperature.

Foam mass composition:

PLA solution Mw = 87 000 g/mol in ethyl acetate 10% w/w.
Plasticizer - glycerol in the amount of 5% w/w as related to polymer solution mass.

Calcium alginate microspheres in the amount of 0.3g per 100 ml foam mass.

The components were mixed in a vessel non-reactive with any of the components. Mixing was effected by means of a stirrer with a flexible shaft. The obtained mass was poured onto a surface of dimensions and shape of the desired foam. Evaporation of PLA solvent - ethyl acetate - took place at ambient temperature for the period of ca. 10 hours, up to the moment that complete, free detachment of the foam product from the substrate was possible.

The presence and kinetics of release of IGF - 1 in vitro was observed using Lowry test menufactured by SERVA Electrophoresis GmbH

Properties of the obtained foam:

Surface mass: 620.03 g/m$^2$,
Free water sorption in time t=8h according to EN13726-1:2005: 3.21 $g_{liquid}/g_{sample}$,
Test liquid A absorbency according to EN13726-1:2005: 3.08 $g_{liquid}/g_{sample}$.

## Claims

1. Medical material containing foam mass composition consisting of a mixture of dibutyrylchitin and polylactide wherein surface mass is 208 g/m$^2$, test liquid A absorbency is $C_A$= 12.748 $g_{liquid}/g_{sample}$ and free water sorption in time t=8h is 10.600 $g_{liquid}/g_{sample}$ and wherein foam mass composition is obtained by mixing a PLA solution in acetone of 5% w/w concentration with a DBC solution in acetone of a 5% w/w concentration with addition of a plasticizer which is glycerol in the amount of 5% w/w as related to the polymer mixture solution mass and a blowing agent which is solid sodium chloride in the amount of 150% w/w as related to polymer mixture solution mass.

2. Medical material according to claim 1 wherein the foam mass composition is permanently y combined with PLA film formed with the melt technique and said combination involves welding the film with the foam at 120°C resulting in a product having surface mass of 350 g/m$^2$ and wherein test liquid A absorbency is $C_A$= 4.60 $g_{liquid}/g_{sample}$, and free water sorption in time t=8h is 5.73 $g_{liquid}/g_{sample}$.

3. Medical material according to claim 1 wherein the foam mass composition is combined with spun-bonded PLA nonwoven and said combination involves welding the film with the foam at 120°C resulting in a product having surface mass of 382 g/m$^2$ and wherein test liquid A absorbency is $C_A$= 3.50 $g_{liquid}/g_{sample}$ and free water sorption in time t=8h is 4.22 $g_{liquid}/g_{sample}$.

4. Medical material containing foam mass composition comprising dibutyrylchitin wherein surface mass is 864.2 g/m$^2$ and free water sorption in time t=8h is 4.3 $g_{liquid}/g_{sample}$ and wherein foam mass composition is obtained by mixing DBC solution in ethanol 8% w/w with addition of a blowing agent which is sodium chloride in amount 100% of polymer solution mass and wherein the obtained mass was poured onto a surface of needled nonwoven made of DBC fibers of 240 g/m$^2$ surface mass.

5. Medical material according to claim 4 wherein said material is subjected to hydrolysis in caustic soda wherein hydrolyzing bath contains 5% of sodium hydroxide (NaOH), hydrolysis is conducted at 25°C for 2 h or less and wherein chitin regenerated from dibutyrylchitin or butyrylchitin esterified to degree between 0 and 2 is obtained.

6. Medical material according to any of the preceding claims for use in manufacturing medical products.

## Patentansprüche

1. Ein medizinisches Material, das eine Schaummassenzusammensetzung enthält, die aus einer Mischung von Dibutyrylchitin und Polylactid besteht, wobei die Oberflächenmasse 208 g/m$^2$, die Testflüssigkeit A Saugfähigkeit $C_A$ =

12,748 $g_{Flüssigkeit}/g_{Probe}$ und die freie Wassersorption in der Zeit t = 8 h 10,600 $g_{Flüssigkeit}/g_{Probe}$ beträgt und wobei die Schaummassenzusammensetzung durch Mischen einer PLA-Lösung in Aceton mit einer Konzentration von 5 Gew.-% mit einer DBC-Lösung in Aceton mit einer Konzentration von 5 Gew.-% unter Zugabe eines Weichmachers, der Glycerin in einer Menge von 5 Gew.-% bezogen auf die Masse der Polymermischungslösung ist, und eines Treibmittels, das festes Natriumchlorid in einer Menge von 150% Gew.-% bezogen auf die Masse der Polymermischungslösung ist, erhalten wird.

2. Das medizinische Material nach Anspruch 1, wobei die Schaummassenzusammensetzung permanent mit einem PLA-Film kombiniert ist, der mit der Schmelzetechnik gebildet wurde, und wobei die Kombination das Verschweißen des Films mit dem Schaum bei 120°C beinhaltet, was zu einem Produkt mit einer Oberflächenmasse von 350 $g/m^2$ führt, und wobei die Testflüssigkeit A Saugfähigkeit $C_A$ = 4,60 $g_{Flüssigkeit}/g_{Probe}$, und die freie Wassersorption in der Zeit t = 8 h 5,73 $g_{Flüssigkeit}/g_{Probe}$, beträgt.

3. Das medizinische Material nach Anspruch 1, wobei die Schaummassenzusammensetzung mit einem spinngebundenen PLA-Vlies kombiniert ist und die Kombination das Verschweißen des Films mit dem Schaum bei 120°C beinhaltet, was zu einem Produkt mit einer Oberflächenmasse von 382 $g/m^2$ führt, und wobei die Testflüssigkeit A Saugfähigkeit $C_A$=3,50 $g_{Flüssigkeit}/g_{Probe}$ und die freie Wassersorption in der Zeit t = 8 h 4,22 $g_{Flüssigkeit}/g_{Probe}$, beträgt.

4. Das medizinische Material, enthaltend eine Schaummassenzusammensetzung, die Dibutyrylchitin umfasst, wobei eine Oberflächenmasse 864,2 $g/m^2$ und die freie Wassersorption in der Zeit t=8 h 4,3 $g_{Flüssigkeit}/g_{Probe}$ beträgt und wobei die Schaummassenzusammensetzung durch Mischen einer DBC-Lösung in Ethanol 8 Gew.-% unter Zugabe eines Treibmittels, das Natriumchlorid in einer Menge von 100% der Masse der Polymermischungslösung ist, erhalten wird, und wobei die erhaltene Masse auf eine Oberfläche eines Nadelvlieses aus DBC-Fasern mit Oberflächenmasse 240 $g/m^2$ gegossen wurde.

5. Das medizinische Material nach Anspruch 4, wobei das Material einer Hydrolyse in Ätznatron unterzogen wird, wobei das Hydrolysebad 5% Natriumhydroxid (NaOH) enthält, die Hydrolyse 2 Stunden oder weniger bei 25°C durchgeführt wird, und wobei das aus Dibutyrylchitin oder Butyrylchitin regenerierte Chitin erhält wird, das zu einem Grad zwischen 0 und 2 verestert ist.

6. Das medizinische Material nach einem der vorhergehenden Ansprüche zur Verwendung bei der Herstellung von medizinischen Produkten.

## Revendications

1. Matériau médical contenant une composition de masse de mousse constituée d'un mélange de dibutyrylchitine et de polylactide dans lequel la masse surfacique est de 208 $g/m^2$, l'absorbance du liquide d'essai A est $C_A$= 12.748 $g_{liquide}/g_{échantillon}$ et la sorption d'eau libre dans le temps t=8h est de 10.600 $g_{liquide}/g_{échantillon}$ et dans lequel la composition de la masse de mousse est obtenue en mélangeant une solution de PLA dans de l'acétone à une concentration de 5% p/p avec une solution de DBC dans de l'acétone à une concentration de 5% p/p avec l'addition d'un plastifiant qui est du glycérol à 5% p/p par rapport à la masse de solution du mélange polymère et un agent gonflant qui est du chlorure de sodium solide en une quantité de 150% p/p par rapport à la masse de solution du mélange polymère.

2. Matériau médical selon la revendication 1, dans lequel la composition de masse de mousse est combinée de façon permanente avec un film en PLA formé avec la technique de fusion et ladite combinaison implique le soudage du film avec la mousse à 120°C résultant en un produit ayant une masse surfacique de 350 $g/m^2$ et dans lequel l'absorbance du liquide d'essai A est $C_A$= 4.60 $g_{liquide}/g_{échantillon}$, et la sorption de l'eau libre en temps t=8h est de 5.73 $g_{liquide}/g_{échantillon}$.

3. Matériau médical selon la revendication 1, dans lequel la composition de masse de mousse est combinée avec un PLA non-tissé filé-lié et ladite combinaison implique de souder le film avec la mousse à 120°C, résultant en un produit ayant une masse surfacique de 382 $g/m^2$ et dans lequel l'absorbance du liquide d'essai A est $C_A$ = 3.50 $g_{liquide}/g_{échantillon}$, et la sorption d'eau libre en temps t=8h correspond à 4.22 $g_{liquide}/g_{échantillon}$.

4. Matériau médical contenant une composition de masse de mousse comprenant de la dibutyrylchitine dans lequel la masse surfacique est de 864.2 $g/m^2$ et la sorption d'eau libre en temps t=8h correspond à 4.3 $g_{liquide}/g_{échantillon}$,

et dans lequel la composition de masse de mousse est obtenue en mélangeant une solution de DBC dans de l'éthanol à 8% p/p avec l'addition d'un agent gonflant qui est du chlorure de sodium en une quantité de 100% de la masse de la solution de polymère et dans lequel la masse obtenue a été coulée sur une surface du tissu non-tissé aiguilleté en fibres DBC de 240 g/m$^2$ de masse surfacique.

5. Matériau médical selon la revendication 4, dans lequel ledit matériau est soumis à une hydrolyse dans de la soude caustique, dans lequel le bain d'hydrolyse contient 5% d'hydroxyde de sodium (NaOH), l'hydrolyse est effectuée à 25°C pendant 2h ou moins et dans laquelle la chitine régénérée à partir de la dibutyrylchitine ou de la butyrylchitine estérifiée à un degré compris entre 0 et 2 est obtenue.

6. Matériau médical selon l'une quelconque des revendications précédentes, destiné à être utilisé dans la fabrication de produits médicaux.

Fig. 1

Fig. 2

Fig. 3

Fig. 4

Fig. 5

Fig. 6

**Fig. 6 cont.**

**Fig. 7**

**Fig. 8**

Fig. 9

Fig. 10

Fig. 11

Fig. 12

Fig. 13

Fig. 14

**Kinetic of releasing of drug into water**

**Kinetic of releasing of drug into saline**

**Fig. 15**

**Fig. 16**

Fig. 17

Fig. 18

**EP 2 814 524 B1**

**REFERENCES CITED IN THE DESCRIPTION**

**Patent documents cited in the description**

- US 2011034575 A
- US 2011031435 A
- WO 2009149845 A
- WO 2011016962 A
- US 2011034571 A
- WO 2011015568 A
- WO 2011015299 A
- EP 1216717 A